# EUROPEAN PATENT APPLICATION

(11) **EP 1 595 568 A1**
(43) Date of publication of application: **16.11.2005**
(21) Application number: 05103758.8
(22) Date of filing: 04.05.2005
(51) Int. Cl.: A61M 25/06

(54) **Needle assembly with tether**

(30) Priority: 10.05.2004 US 842593
(71) Applicant: Becton, Dickinson and Company, Franklin Lakes, New Jersey 07417-1880 (US)
(72) Inventor: Howell, Glade H., Sandy, Utah 84094 (US); Adams, Chad M., Cedar Hills, Utah 84062 (US); Harding, Weston F., Lehi, Utah 84043 (US); Cindrich, Chris, Drapre, Utah 84020 (US)
(74) Representative: Weber, Thomas

(57) **Abstract**

A needle assembly with needle shield that is connected to the needle hub by a tether. The tether prevents unwanted proximal movement of the needle with respect to the needle shield once the needle has been withdrawn into the needle shield. The tether is maintained in its compressed state by use of the invention for ease of assembly,

## Description

### FIELD OF THE INVENTION

The present invention relates to the field of shielding tips of needles and the like. Specifically, aspects of the invention relate to improvements in deployment and retention of tethered needle shields used in a needle assembly, such as those used in connection with the insertion of intravenous catheters that will safely shield the sharp distal tip of the needle after the needle has been used on a patient.

### BACKGROUND OF THE INVENTION

Catheters, particularly intravenous (IV) catheters, are used for infusing fluid, such as normal saline solution, various medicaments and total parenteral nutrition, into a patient or withdrawing blood from a patient. Peripheral IV catheters tend to be relatively short, and are on the order of about one and one-half inches in length. The most common type of IV catheter is an over the needle peripheral IV catheter. As its name implies, an over the needle catheter is mounted over an introducer needle having a sharp distal tip. The catheter and the introducer needle are assembled so that the distal tip of the introducer needle extends beyond the distal tip of the catheter with the bevel of the needle facing up away from the patient's skin.

The catheter and introducer needle assembly is inserted at a shallow angle through the patient's skin into a peripheral blood vessel. These are smaller blood vessels that are not connected directly to the heart but are the branches of the central blood vessels that are directly connected to the heart. In order to verify proper placement of the assembly in the blood vessel, the clinician confirms that there is flashback of blood in the needle and in a flashback chamber located at the proximal end of the needle. The flashback chamber is typically formed as part of the needle hub. Once proper placement is confirmed, the clinician applies pressure to the blood vessel by pressing down on the patient's skin over the distal tip of the introducer needle and the catheter. This finger pressure occludes further blood flow through the introducer needle. The clinician withdraws the introducer needle, leaving the catheter in place, and attaches a fluid-handling device to the catheter hub.

Once the introducer needle is withdrawn from the catheter, it is a "blood contaminated sharp" and should be properly handled. In recent years, there has been great concern over the contamination of clinicians with a patient's blood and a recognition that "blood contaminated sharps" should be immediately disposed. This concern has arisen in part because of the advent of currently incurable and fatal diseases, such as Acquired Immune Deficiency Syndrome ("AIDS") and hepatitis, which can be transmitted by the exchange of body fluids from an infected person to another person. Thus, contact with the body fluid of an AIDS or hepatitis infected person should be avoided to prevent the transmission of such diseases to a healthy person. As noted above, if an introducer needle has been used to place a catheter in the vein of an AIDS, or hepatitis, infected person, the introducer needle can be a vehicle for the transmission of the disease. Although clinicians are aware of the need to properly handle "blood contaminated sharps," unfortunately, in certain medical environments, such as emergency situations or as a result of inattention or neglect, needlesticks with contaminated introducer needles still occur.

To help reduce the risks and inconvenience arising from of accidental needlesticks by "blood contaminated sharps," various needle shields have been developed. Generally, such needle shields work for their intended purpose but could be improved. For example, some needle shields are not positively connected to the introducer needle assembly and could be advanced distally past the distal end of the introducer needle. In addition, some introducer needle assemblies having a needle shield associated therewith are bulky, difficult to use or require special features or techniques to be operative or are ergonomically uncomfortable for the clinician to use.

In the past, improvements to needle shields have been made, such as in US Patent Nos. 6,234,999 and 6,425,884 both to Wemmert et al., and 6,527,747 to Adams et al. each incorporated herein by reference in its entirety. It is desirable to have a needle shield assembly including a tether that is readily assembled and deployed without difficulty or interferences with the preferred clinician techniques.

### SUMMARY OF THE INVENTION

In accord with one aspect of this invention, a needle assembly providing a needle shield that is positively connected to the needle assembly to prevent the needle shield from being advanced distally past the distal end of the needle.

It is yet another aspect of this invention to provide a needle assembly with a needle shield that is simple and easy to use.

It is still another aspect of this invention to provide a needle assembly with a needle shield that requires no special features or technique to be operative.

It is a further aspect of this invention to provide a needle assembly with a needle shield that is ergonomically comfortable for the clinician to use.

It is yet another aspect of this invention to provide a needle assembly with a tether that is easy to manufacture and assemble.

In accord with one implementation of this invention, the needle assembly includes a needle shield connected to the needle hub by a tether. The needle has a sharp distal tip and a proximal end connected to the distal end of a needle hub.

The needle shield is located substantially between the catheter and the needle hub and defines a longitudinally extending passage through which the needle extends. The needle shield may include some barrier or other mechanism that prevents the sharp distal tip of the needle from being moved distally with respect to the needle shield after the sharp distal tip of the needle has been withdrawn into the needle shield. This prevents the sharp distal tip of the needle from being re-exposed once it has been withdrawn into the needle shield.

The needle shield is connected to the needle hub by a tether that prevents the needle shield from being moved distally past the sharp distal tip of the needle, once the needle has been proximally withdrawn into the needle shield. The combination of the barrier or other mechanism associated with the needle shield and the tether prevents distal movement of the needle with respect to the needle shield. This ensures that the sharp distal tip of the needle remains trapped in the needle shield after the needle has been withdrawn proximally into the needle shield after use.

In one particular design of tethered needles, the tether is folded over itself to form a plurality of pleats like an accordion. Optionally, each pleat or fold may define a central opening therein to allow the needle to extend through each pleat or fold no matter whether the tether is completely extended or completely folded with the needle shield adjacent to the needle hub. In another design of tethered needles, the tether is constructed of a compressible material, and is compressed between the hub and shield. It is desired that the size of the overall device be minimized, therefore, the tether is designed to fit into a small space, and as a result the tether has a tendency to spring apart due to its compression. The tether, when sprung apart, is difficult to integrate into the assembly process.

One embodiment of the present invention utilizes a capture feature on the housing of either the hub or the shield to capture the tether such that it is easy to assemble. In this embodiment of the invention, the needle assembly, comprises a needle having a proximal end and a distal end; a needle hub having a proximal end and a distal end connected to the proximal end of the needle; a needle shield slidingly disposed about the needle; a compressible tether securely connecting the needle hub to the needle shield; a member is adapted to maintain the tether in a compressed condition disposed on the shield or the hub operably engaged to the tether. Optionally, the tether is formed from a plurality of pleats. Optionally, the plurality of pleats each contains a hole through which the needle extends. Optionally, the member is at least one latch, which engages the tether in a compressed condition. Optionally, the latch engages the pleats when the tether is in a compressed condition. Optionally, the member is a pin, which engages at least one recess disposed on the pleats when the tether is in a compressed condition.

In another embodiment of the invention, a tie down is used to capture the tether to either the hub or the shield or to itself, such that it is easy to assemble. In this embodiment of the invention, a feature on the tether itself prevents the tether from springing apart, making the tether easy to assemble. In this embodiment of the invention, the needle assembly comprises a needle having a proximal end and a distal end; a needle hub having a distal end connected to the proximal end of the needle and a proximal end; a needle shield disposed about the needle; a compressible tether connecting the needle hub to the needle shield, a member located on the tether adapted to maintain the tether in a compressed condition. Optionally, the tether is formed from a plurality of pleats. Optionally, the plurality of pleats each contains a hole through which the needle extends. Optionally, the member is a portion of the pleat that creates interference between the pleats. Optionally, the member is a frangible strap, connector, or connection.

In the case of folded tethers, another embodiment of the invention is to reduce the spring bias of the folds such that the tether no longer has a bias to spring apart. One method of reducing the spring bias of the folds is to remove material in the fold such that the biasing force of the fold is insufficient to move the tether. Another method of reducing the spring bias of the folds is to construct the fold in such a way that the fold does not bias the tether to unfold, but to have a neutral bias or a bias in the folded direction. In this embodiment of the invention, the needle assembly comprises a needle having a proximal end and a distal end; a needle hub having a proximal end and a distal end connected to the proximal end of the needle; a needle shield disposed about the needle; a pleated tether formed from a plurality of pleats joined by a crease, with a hole that is defined in at least one crease connecting the needle hub to the needle shield. Optionally, the plurality of pleats each contains a hole through which the needle extends.

In still yet another alternate embodiment of the invention, the needle assembly comprises a needle having a proximal end and a distal end; a needle hub having a proximal end and a distal end connected to the proximal end of the needle; a needle shield disposed about the needle; a pleated tether formed from a plurality of pleats joined by a crease connecting the needle hub to the needle shield. Optionally, each crease is formed by more than one hinge. Optionally, the plurality of pleats each contains a hole through which the needle extends.

In another embodiment of the invention, an adhesive is used with the tether to prevent the tether from springing apart. In this embodiment of the invention, the needle assembly comprises a needle having a proximal end and a distal end; a needle hub having a proximal end and a distal end connected to the proximal end of the catheter; a needle shield disposed about the needle; a tether capable of being folded formed from a plurality of pleats, connecting the needle hub to the needle shield. Optionally, an adhesive which resists un-folding of the tether is applied to at least a portion of the tether. Optionally, the plurality of pleats each contains a hole through which the needle extends. Optionally, the adhesive is applied to at least one edge of the pleats. Optionally, the adhesive is applied to at least one face of the pleats.

These and other aspects and advantages of the present invention in its various implementations will become apparent from the subsequent detailed description of the preferred embodiment and the claims taken in conjunction with the accompanying drawings.

### BRIEF DESCRIPTION OF THE DRAWINGS

The preferred embodiments are illustrated in the drawings in which like reference numerals refer to like elements and in which:

FIG. 1 is a perspective view of a ported catheter and introducer needle assembly with the needle shield, tether retainer and tether of the present invention prior to activation.

FIG. 2 is a top plan view of a ported catheter and introducer needle assembly with the needle shield, tether retainer, and tether of the present invention prior to activation.

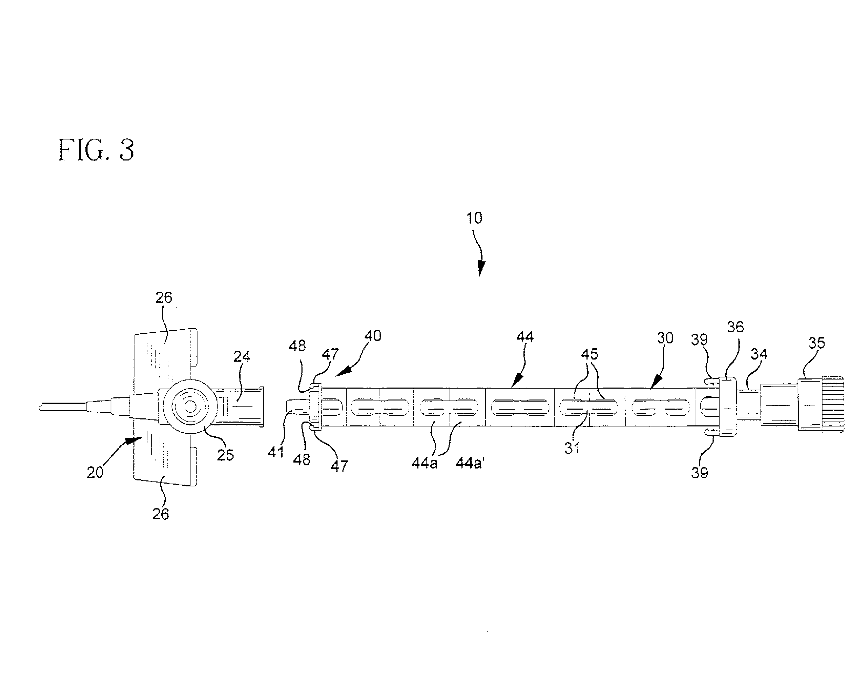

FIG. 3 is a top plan view of a ported catheter and introducer needle assembly with the needle shield, tether retainer, and tether of the present invention, after the needle has been withdrawn from the catheter into the needle shield and the needle shield has been removed from the catheter hub.

FIG. 4 is a cross sectional view of a portion of the introducer needle assembly showing the needle shield, tether retainer, and tether prior to activation.

FIG. 5 is a bottom perspective view in partial cross section and partially in phantom of a portion of the needle shield, the tether, the tether retainer, and the needle hub illustrating one method of connecting the tether to the needle shield and the needle hub.

FIG. 6 is an alternate embodiment of the invention shown in a cross sectional view of a portion of the introducer needle assembly showing the needle shield, the tether retainer, and tether prior to activation.

FIG. 7 is a top plan view of a tether of the present invention.

FIG. 8 is a cross sectional side view along 8-8 of the tether of FIG. 7.

FIG. 9 is a top plan view of another embodiment of the tether.

FIG. 10 is a top plan view of another alternate embodiment of the tether.

FIG. 11 is a cross sectional side view along 11-11. of the tether of FIG. 10.

FIG. 12 is a top plan view of another alternate embodiment of the tether.

FIG. 13 is a cross sectional side view along 13-13 of the tether of FIG. 12.

FIG. 14 is a top plan view of another alternate embodiment of the tether.

FIG. 15 is a cross sectional side view along 15-15 of the tether of FIG. 14.

### DETAILED DESCRIPTION OF THE INVENTION

As used herein, the term "proximal" refers to a location on the device that is closest to the clinician using the device and farthest from the patient in connection with whom the device is used when the device is used in its normal operation, Conversely, the term "distal" refers to a location on the device that is farthest from the clinician using the device and closest to the patient in connection with whom the device is used when the device is used in its normal operation.

As used herein, the term "top", "up" or "upwardly" refers to a location with respect to the device that, during normal use, is radially away from the longitudinal axis of the device and away from the patient's skin. Conversely, as used herein, the term "bottom", down" or "downwardly" refers to a location with respect to the device that, during normal use, is radially away from the longitudinal axis of the device and toward the patient's skin.

As used herein, the term "in" or "inwardly" refers to a location with respect to the device that, during normal use, is toward the inside of the device. Conversely, as used herein, the term "out" or "outwardly" refers to a location with respect to the device that, during normal use, is toward the outside of the device.

The catheter and introducer needle assembly is identified generally by the numeral 10. It includes a catheter assembly 20 and an introducer needle assembly 30 that includes a needle shield 40 and a tether 44. It will be appreciated that certain aspects of the invention may be implemented with other than catheter and introducer needles assemblies such as those disclosed in U.S. Application Serial number 09/717,148 filed November 21, 2000, herein incorporated by reference in its entirety. Furthermore, other aspects of the invention may be implemented with other types of needles, such as anesthesia needles, biopsy needles, syringe needles, pen needles or needles for blood collection sets.

Catheter assembly 20 includes a catheter 21 that has a proximal end and a distal end. A catheter adapter 24 is affixed to the proximal end of catheter 21. Suitable materials for catheter 21 include, but are not limited to, thermoplastic resins such as polytetrafluoroethylene (PTFE), fluorinated ethylene propylene copolymer (FEP), polyurethane and the like. Preferably, catheter 21 is formed from thermoplastic hydrophilic polyurethane that softens with exposure to physiological conditions present in the patient's body. Suitable materials for catheter adapter 24 include, but are not limited to, thermoplastic polymeric resins such as polycarbonate, polystyrene, polypropylene and the like.

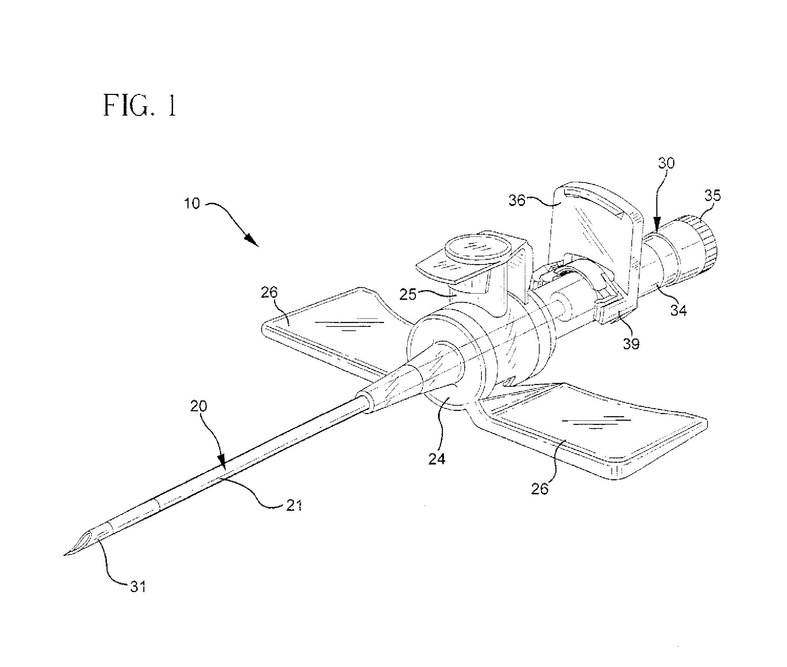

Catheter adapter 24 may include a radially outwardly extending side port 25, which is useful for connecting a fluid-handling device to catheter 21 for infusing fluids into the patient's blood vessel. See FIG. 1. Preferably, side port 25 extends upwardly away from the patient when catheter 21 is inserted into the patient. In addition, a pair of wings 26 may be attached to or integrally formed with catheter adapter 24. Wings 26 are useful to stabilize catheter 21 in the patient and provide a surface that facilitates taping of catheter assembly 20 to the patient to fix catheter 21 properly in the patient's vasculature.

Although, the majority of the description of catheter and introducer needle assembly 10 of this invention is directed to a catheter having a side port, it is to be understood that aspects of this invention can also be used with a straight catheter, syringes, introducers, long needles (such as anesthesia, biopsy, spinal and epidural needles), blood collection sets, pen needles, fluid transfer needles, or any device including a sharp needle-like point.

A cover 27 may be used to cover catheter 21 and the introducer needle 31 prior to use. Preferably cover 27 is formed from a hard polymeric material such as thermoplastic polymeric resins, which include polycarbonate, polystyrene, polypropylene and the like. Of course other materials may also be used for cover 27.

Introducer needle assembly 30 includes introducer needle 31 having a sharp distal tip defined by a bevel. A notch may be included in the wall of the needle to provide flashback visualization through the catheter wall, if desired. The proximal end of introducer needle 31 is connected to a needle hub 34. Introducer needle 31 is preferably formed from stainless steel. Needle hub 34 can include an integrated flashback chamber having an open proximal end. Needle hub 34 is preferably formed from the same types of materials that are used to form catheter adapter 24. Preferably, the open proximal end of needle hub 34 is closed to fluid flow by a vented plug 35, which allows air but not fluid to flow therethrough. This facilitates blood flow into the flashback chamber but prevents blood from leaking out.

Vented plug 35 may be removably connected to the open proximal end of needle hub 34, This allows a clinician to gain access to the patient's blood vessel during venipuncture. In this way, a syringe could be attached to the open proximal end of needle hub 34 for aspiration during insertion of catheter and introducer needle assembly 10.

Needle hub 34 may also include a radially extending tab 36, which may be grasped in combination with side port 25 to facilitate insertion of assembly 10 into the patient. Tab 36 may also be useful for withdrawing introducer needle 31 from catheter 21 after catheter 21 has been properly located in the patient's vasculature.

In addition, needle hub 34 may include a pair of longitudinally extending fingers 39. These fingers 39 frictionally engage needle shield 40. In this way, needle hub 34 is held in place adjacent to catheter hub 24 so that the sharp distal tip of introducer needle 31 extends distally of the distal end of catheter 21. The proximally directed force needed to overcome the frictional engagement between fingers 39 and needle shield 40 is less than the proximally directed force needed to overcome the removable connection between needle shield 40 and catheter adapter 24.

As noted above, introducer needle assembly 30 also includes needle shield 40. Like catheter adapter 24 and needle hub 34, suitable materials for needle shield 40 include, but are not limited to, thermoplastic polymeric resins such as polycarbonate, polystyrene, polypropylene and the like. Needle shield 40 includes main body portion 41, which defines a longitudinally extending passage 42 therethrough. Longitudinally extending passage 42 allows introducer needle 31 to extend longitudinally through main body portion 41. The diameter of longitudinally extending passage 42 is slightly larger than the diameter of the main portion of introducer needle 31. This allows the main portion of introducer needle 31 to easily pass through longitudinally extending passage 42.

An annular lip 43 is located along the distal portion of longitudinally extending passage 42. Preferably, lip 43 is integrally formed with needle shield 40 and contacts the main portion of introducer needle 31. In this way, lip 43 acts as a fluid seal along the main portion of introducer needle 31. During venipuncture, blood flow between introducer needle 31 and needle shield 40 is minimized to minimize blood leakage from the device.

A transverse barrier 49 may be located in needle shield 40 to act as a barrier against re-exposure of the sharp distal tip of introducer needle 31 after it has been withdrawn into main body portion 41. Transverse barrier 49 is preferably formed as an integral member from a resilient metal, such as stainless steel. Preferably, transverse barrier is formed as a generally cylindrical main body portion 49b with two lips 49a in the configuration of a duckbill extending generally proximally from the distal end of main body portion 49b. The longitudinal dimension of main body portion 49b and lips 49a can be any value but preferably, main body portion 49b is longer than lips 49a. This relationship facilitates the manufacture of this device and makes for a more robust design.

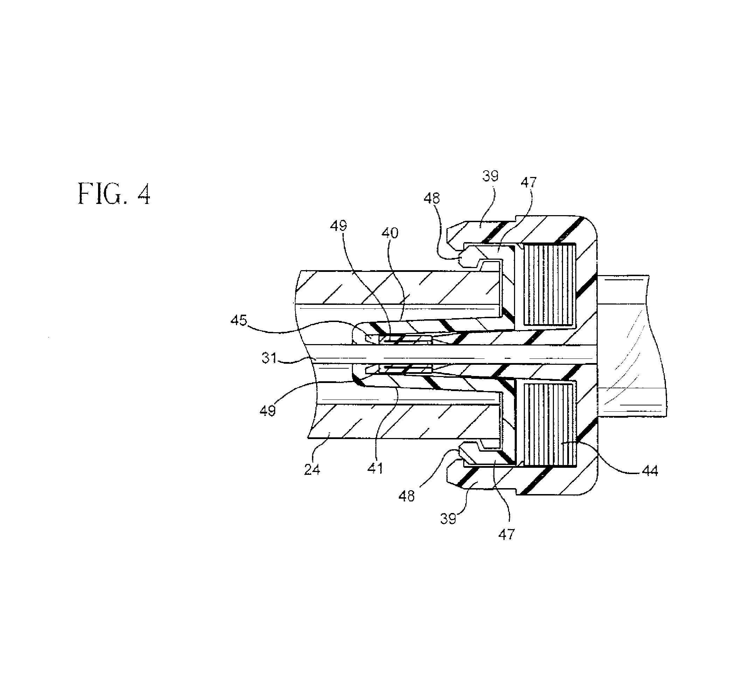

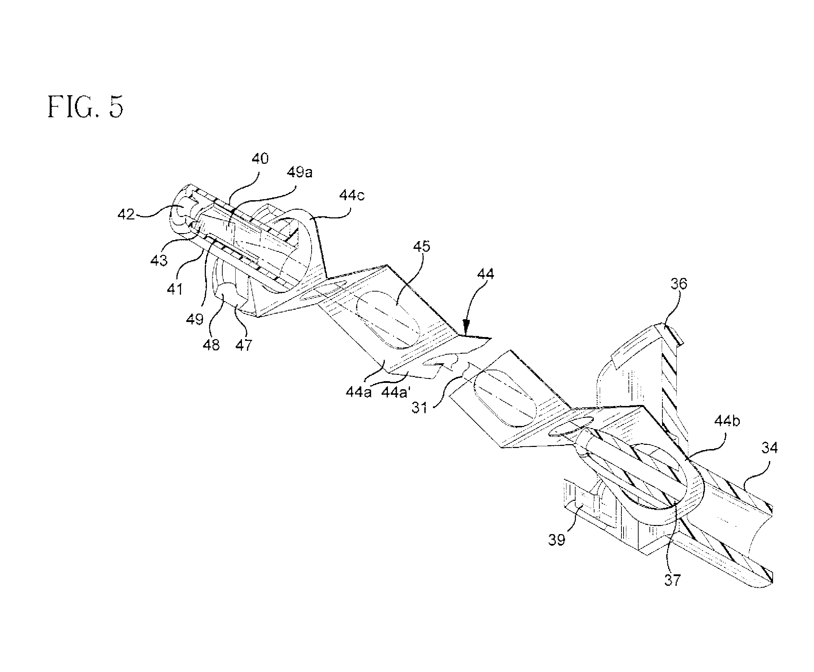

Prior to use, lips 49a extend toward the proximal end of introducer needle 31 and generally parallel to the main body portion of introducer needle 31. See FIG. 4. After proper placement of catheter 21 into a patient's blood vessel, lips 49a ride along the main body portion of introducer needle 31 when introducer needle 31 is being withdrawn from catheter 21. Once the sharp distal tip of introducer needle 31 is withdrawn to a position proximal of lips 49a, the resilient nature of lips 49a causes them to return to a position that is transverse to introducer needle 31. The configuration of lips 49a as shown in FIG. 5 prevents re-exposure of the sharp distal tip that could occur if introducer needle 31 were thereafter moved distally with respect to needle shield 40. See FIG. 5.

Other types of transverse barriers could also be used. For example, a transverse barrier having a single arm could be used in place of the pair of lips 49a forming a duckbill as described above.

Main body portion 41 also includes a plurality of longitudinally extending fingers 47. Fingers 47 engage catheter adapter 24 to hold introducer needle assembly 30 together with catheter assembly 20. Thus, as needle hub 34 is moved proximally with respect to catheter adapter 24, needle shield 40 remains adjacent to catheter adapter 24. As described in more detail below, tether 44 has an unfurled or extended length that maintains the sharp distal tip of introducer needle 31 in main body portion 41 of needle shield when tether 44 is fully extended. Thus, once the sharp distal tip of introducer needle 31 is moved into main body portion 41 of needle shield 40, any additional proximally directed movement applied to needle hub 24 will overcome the friction force between fingers 47 and catheter adapter 24. In this way, needle shield 40 can be removed from catheter adapter 24. If desired, main body portion 41 may be configured so that the exterior surface of the distal portion of main body portion 41 frictionally engages the interior surface of catheter adapter 24 to help hold needle shield 40 with catheter adapter 24.

Fingers 47 may also include radially inwardly directed projections 48. Projections 48 are sized to mechanically engage catheter adapter 24 by a snap fit. If projections 48 are used, the force needed to remove main body portion 41 from catheter adapter 24 may be more precisely controlled than if only fingers 47 or a friction fit are used. Once the proper proximally directed force is applied to main body portion 41, projections 48 override catheter adapter 24 and main body portion 41 can be removed from catheter adapter 24. Again, tether 44 maintains main body portion 41 in the proper location so that the sharp distal tip of introducer needle 31 is safely located within main body portion 41 of needle shield 40.

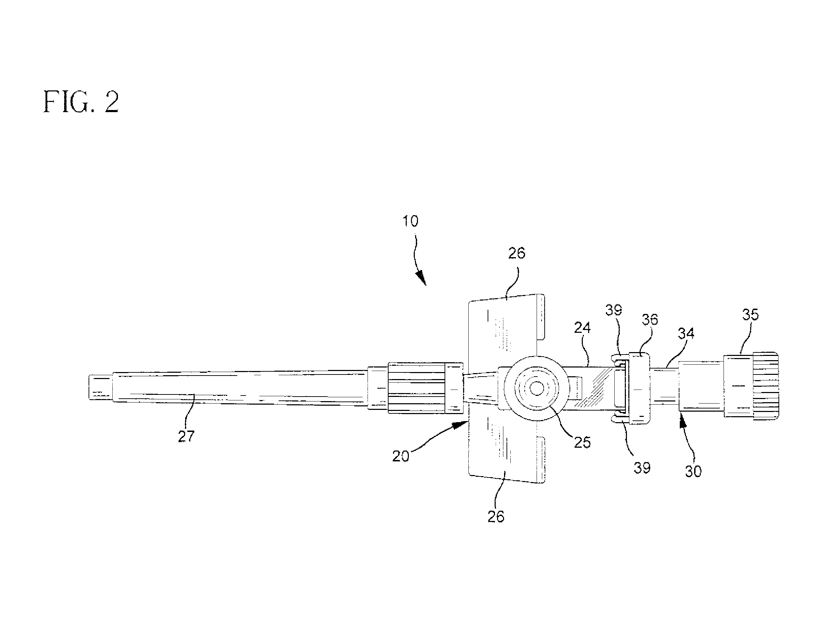

Tether 44 has a length that is selected to the exposed length of introducer needle 31. This length maintains the sharp distal tip of introducer needle 31 in main body portion 41 of needle shield 40 when tether 44 is fully extended. The length of the tether is selected by determining the needle length, shield length, and the tether stretch when shield disconnects from catheter adapter. Tether 44 is folded over itself to form a plurality of pleats 44a like an accordion. Each pleat 44a defines a central opening 45 therein to allow introducer needle 31 to extend through each pleat 44a whether tether 44 is completely extended, see FIG. 4, or completely folded with main body portion 41 of needle shield 40 adjacent to needle hub 34, see FIGS. 2 and 4. By forming tether 44 in this way, a compact design is achieved for catheter and introducer needle assembly 10.

Tether 44 can be made of any relatively stiff yet flexible material. However, polyethylene terephthalate (PET) is the preferred material. One drawback of using PET is that it is relatively stiff so that when it is folded into a pleated or an accordion-like configuration, it provides a slight biasing force to help maintain tether 44 in the completely extended position.

This biasing force causes some difficulty in assembly, since tether 44 tends to bias apart during assembly. One embodiment of an aspect of the invention utilizes a releasable-retention member on hub 34 to retain tether 44 after tether 44 has been assembled onto hub 34. An exemplary configuration of this member is tang 50, shown in FIGs. 4 & 5. As shown in the figure, tang 50 is a slight radial protrusion along the interior portion of finger 39, which minimally interferes with a portion of tether 44, in such a fashion that tether 44 is detachably retained on hub 34 after assembly. Tang 50 interferes with an outer dimension of tether 44. The minimal interference of tang 50 and tether 44 is sufficient to prevent the biasing force of tether 44 from moving tether 44 distally away (unfurling) from hub 34, yet when additional distal force is applied to tether 44, tether 44 is easily deployed from hub 34. The folded tether can now be assembled to hub 34 without tending to unfold. The sub-assembly consisting of hub 34 including tang 50 and folded tether 44 then may be incorporated into the assembly process as a stable sub-unit, which aids in manufacturability of overall assembly 10. Optionally, tang 50 is constructed in a ratchet-type configuration so that tang 50 does not interfere with the assembly of tether 44 onto hub 34 and minimally interferes with the deployment of tether 44 during operation of the overall device 10. In the ratchet-type configuration, tang 50 is shaped as a protrusion from an interior portion of finger 39 such that tang 50 has a tapered portion leading to a proximal face which contacts a distal portion of tether 44 to retain tether 44 within hub 34. Alternatively, tang 50 is a longitradinal protrusion along the interior portion of finger 39, which frictionally interacts by virtue of a dimensional interference fit with an exterior portion of tether 44. Alternatively, tang 50 is located on post 51 with a similar configuration; however, in this case tang 50 would interact with opening 45 in tether 44, rather than an exterior portion. Alternatively, tether 44 could be detachably retained to shield 40, in which case the releasable-retention member would be located on shield 40. Alternatively, the releasable-retention member is a longitudinal pin projecting distally from a distal face of hub 34, which frictionally interacts with an exterior portion of tether 44, preferably interacting with a corresponding recess in tether 44. Although it is preferred to have the releasable-retention member in the form of a tang 50 interfering with tether 44, it is to be understood that other well-known configurations for temporary article retention may be used, such as latches, catches, pins, friction fits, tack welding, spot adhesive placement,

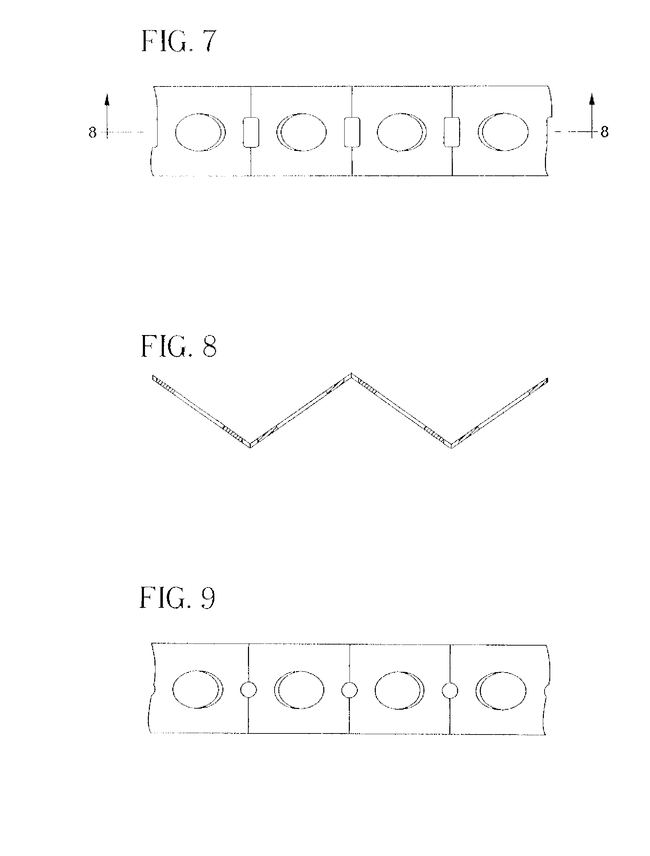

In another embodiment of the invention, shown in FIGs. 7 & 8, tether 44 further comprises secondary opening 65 at each fold 60 of each pleat 44a of tether 44. Secondary opening 65 reduces the biasing force of tether 44 by removing portions of fold 60. The removal of material from fold 60 has the effect of reducing the overall spring rate of each individual fold 60, thereby reducing the overall spring rate of the folded tether. Thus, the tendency of tether 44 to spring apart when folded is reduced. The folded tether can now be assembled to hub 34 without tending to unfold. The sub-assembly consisting of hub 34 and folded tether 44 then may be incorporated into the assembly process as a stable sub-unit, which aids in manufacturability of overall assembly 10. Alternatively, tether 44 may be assembled onto shield 40 to create a stable sub-unit. Secondary opening 65 may also be of any shape as shown in FIG. 9, in which secondary opening 65' is a circular opening. Preferably, secondary opening 65 does not significantly reduce the tensile strength of tether 44. Optionally secondary opening 65 may consist of multiple openings, such as in the case of perforations.

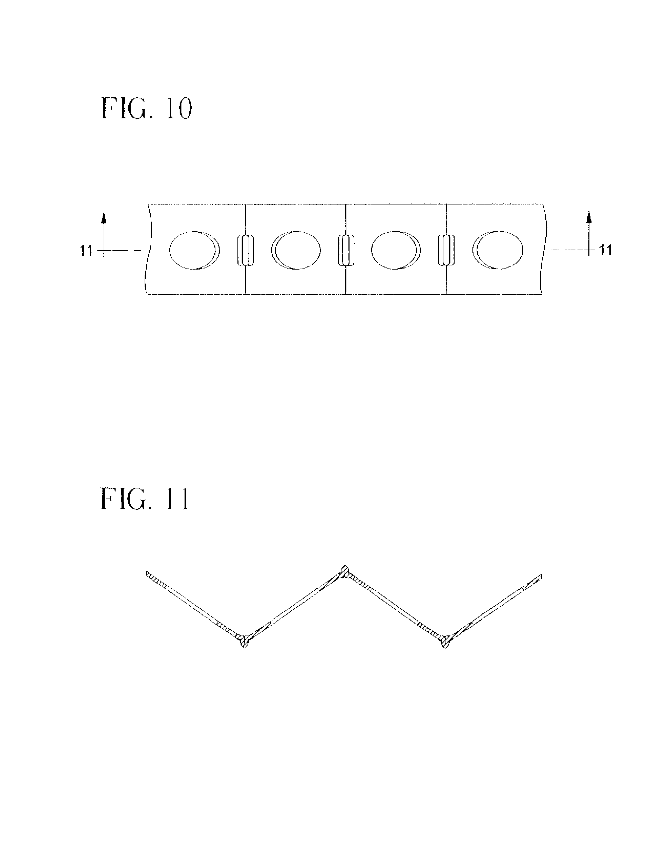

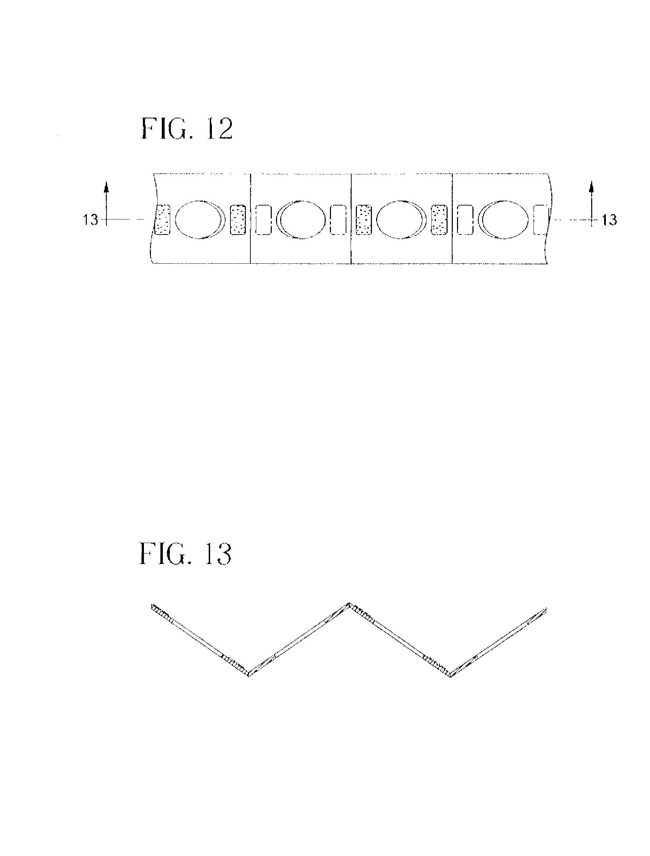

In an alternate embodiment of the invention, as shown in Fig. 10 & 11, fold 60 is constructed such that additional material is used to create crease 70. Therefore, crease 70 is more than just a simple fold with a single hinge, and can be fabricated in tether 44 with a variety of well know web-type processes, such as double folding, triple folding, embossing, stamping, gathering, pinching, gusseting, or thermoforming. Crease 70 has the effect of reducing the overall spring rate of fold 60, thereby reducing the overall spring rate of the folded tether. Thus, the tendency of tether 44 to spring apart when folded is reduced. Preferably, crease 70 is fabricated in such a way that crease 70 does not reduce the tensile strength of tether 44. As in the previous embodiment, the folded tether can now be assembled to shield 40 or hub 34 without tending to unfold. Optionally, secondary opening 65 may be utilized to further reduce the spring rate of tether 44.

In another embodiment of the invention, tether 44 may be fabricated with a releasable-retention mechanism consisting of portions of pleats 44a of tether 44 which interfere with other portions of pleats 44a, such that when tether 44 is folded pleats 44a of tether 44 tend to stick together. As shown in the FIG. 12 a portion of pleat 44a defined as first portion 85 interacts with second portion 86 to resist the bias of tether 44 after tether 44 has been folded. When tether 44 is folded first portion 85 engages second portion 86 in such a fashion that tether 44 tends to stay folded, yet tether 44 becomes easily unfolded when deployed in the use of the device 10. First portion 85 may be an embossed or raised area, while second portion 86 may be a mating depressed area. The portions 85,86 along the faces of at least 2 pleats 44a interact so that the there is some resistance to unfolding of tether 44. The interference of portions 85,86 along all the pleats 44a of the tether are designed such that the force to remove first portion 85 from second portion 86 is greater than the biasing force of the hinge, which tends to unfurl tether 44. Alternatively, a similar design having the same functional result is a first portion 85 comprising an embossed raised area and second portion 86 is a corresponding aperture in tether 44. Although it is preferred to have the releasable-retention member in the form of an embossed area interfacing with a depressed area or aperture, it is to be understood that other well-known configurations for temporary article retention may be used, such as hook & loops, friction fits, and tack welding. In an alternate embodiment consistent with portions of the tether interacting with itself, a portion of tether 44 is formed into a frangible strap, which, after tether 44 is folded, is attached from the distal most pleat of tether 44 to the proximal most pleat of tether 44. The frangible strap may be tack welded to either distal or proximal pleat 44a of tether 44. The length of the frangible strap is approximately the overall height of the folded tether, thereby preventing tether 44 from unfurling. As tether 44 is deployed in the device, the frangible strap is fractured and tether 44 is able to unfurl to the full length of tether 44.

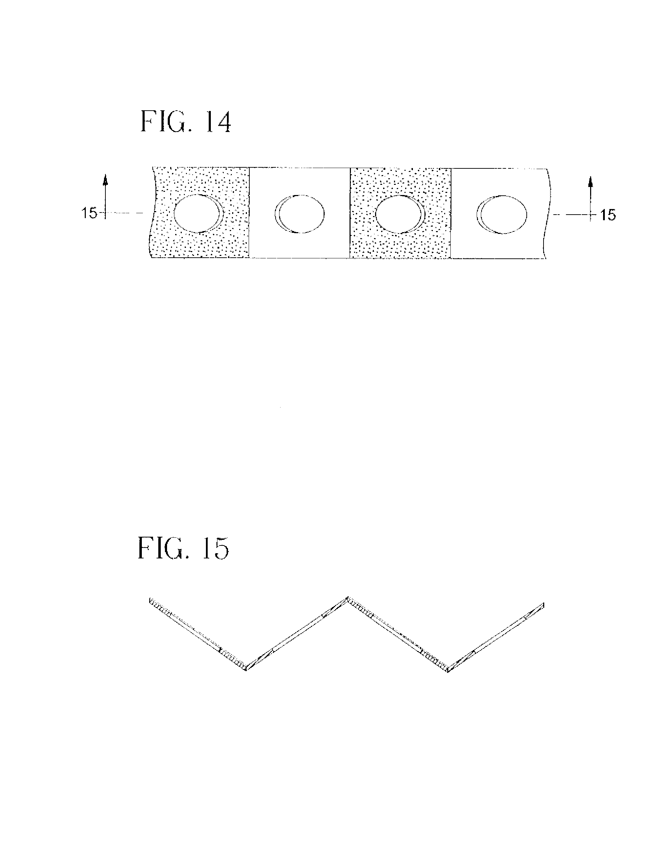

In yet another embodiment of the invention, a releasable adhesive is used to maintain tether 44 in a compressed state. As shown in FIGs. 14 & 15, adhesive 95 is place on a portion of tether 44 such that adhesive 95 maintains tether 44 in a compressed state. As shown in FIG. 14 a portion of pleat 44a is covered by adhesive 95 which releasably bonds to pleat 44a and facing pleat 44a' to resist the bias of tether 44 after tether 44 has been folded. When tether 44 is folded, adhesive 95 engages second pleat 44a' in such a fashion that tether 44 tends to stay folded, yet tether 44 becomes easily unfolded when deployed in the use of the device 10. The faces of at least 2 pleats 44a are temporarily bonded so that the there is some resistance to unfolding of tether 44. The adhesive bonding force is selected such that the force to remove first portion 85 from second portion 86 is greater than the biasing force of the hinge, which tends to unfurl tether 44. Such releasable adhesives are well known in the art. Although it is preferred to cover the entire face of pleat 44 with adhesive 95, it is to be understood that a smaller portion of the tether may be covered with adhesive, such as a portion of a pleat (such as depicted in FIG.12) or covering pleat edge 96 with adhesive, achieving similar functional results.

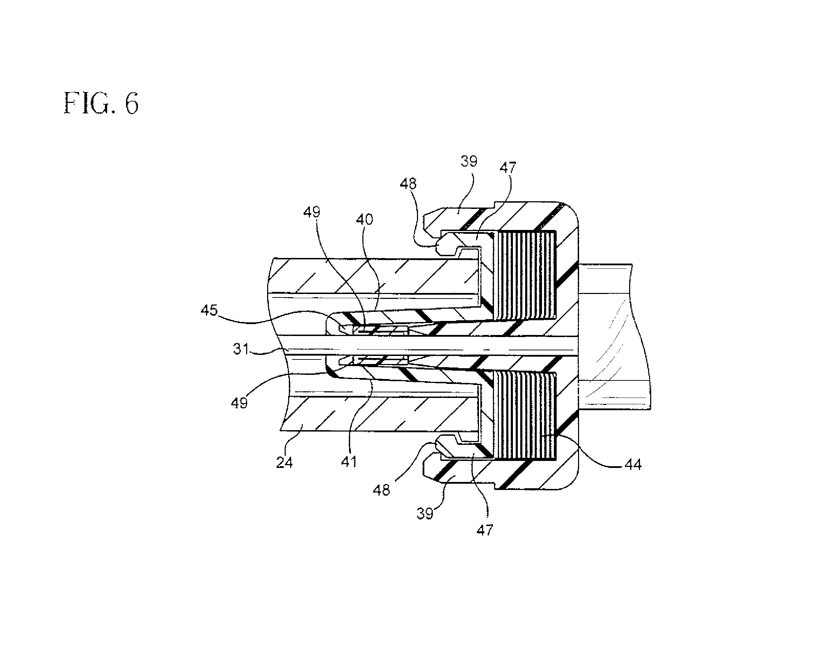

Each opening 45 in pleats 44a should be of the smallest: size practicable to allow introducer needle 31 to extend therethrough. By forming each opening 45 accordingly, a sufficient amount of the material forming tether 44 extends between adjacent openings 45 and between each opening 45 and the nearest edge to tether 44. In this way, the tensile strength of tether 44 is not compromised. Preferably, the configuration of each opening 45 is such that the longitudinal dimension A is greater than the radial dimension B. See, e.g. FIGS. 7-15. This configuration can take the specific form of a racetrack, i.e. where the sides of each opening 45 are straight and are joined by a curved surface at both ends. Alternatively, each opening 45 can have an elliptical configuration. Each opening 45 could also be generally rectangular configuration. In addition, each opening 45 could have a different specific shape so long as the shape is narrower in the radial direction than it is in the longitudinal direction. As an example, each opening 45 as shown in FIG. 7 could have a radial dimension of 0.108 inches and a longitudinal dimension of 0.138 inches. With these dimensions, tether 44 should be 0.250 inches wide and preferably 0.0014 inches thick. Openings 45 Should be aligned along the longitudinal axis of tether 44 and should be about 0.100 inches apart with the center to center distance between each opening 45 about 0.250 inches apart. In an alternate embodiment of the invention, as shown in FIG. 6, openings 45 are constructed such that they provide an interference fit with post 51 on hub 34. The interference of opening 45 and post 51 is in at least one radial portion of opening 45, such that opening 45 resists the insertion or removal of post 51. The interference fit is designed such that the frictional forces required to move pleat 44a longitudinally along post 51 is greater than the biasing force of tether 44. Optionally, to aid in assembly and molding, post 51 may be tapered such that the distal radial dimension of post 51 is smaller that the proximal radial dimension of post 51. The interference of post 51 with tether 44 overcomes the bias of tether 44, and the tendency of tether 44 to spring apart. In this way tether 44 may be pre-assembled onto hub 34 to create a sub unit, which aids in the manufacturability of overall assembly 10.. Alternatively, the opening in the pleat adjacent to the distal pleat may be sized to frictionally engage the post while the remaining pleat openings are sized larger than the diameter of the post. This will facilitate compression of the tether but allow for easy removal of the tether from the post during needle shielding.

In another embodiment of the invention, the tether may be assembled onto the needle hub and then compressed with a tool that will apply heat and pressure. The heat and pressure modify the spring rate of the tether such that the tether tends to remain compressed. In this manner the material of the tether will take a 'set' in a compressed shape until the tether is deployed during the catheter insertion procedure.

Tether 44 can be connected to needle hub 34 and needle shield 40 by any standard means, such as by an adhesive or by heat-sealing. Preferably, tether 44 is connected to needle hub 34 and needle shield 40 through mechanical engagement as well as by an adhesive. For example, a slot 37 may be formed along a distal portion of needle hub 34 where one end of tether 44 can be placed. Slot 37 thus provides an edge along which a proximal pleat 44b of tether 44 can mechanically engage needle hub 34 to prevent tether 44 from being removed from needle hub 34 during use. See FIG. 5. Alternatively, proximal pleat 44b and/or distal pleat 44c could be integrally molded to either the hub or shield, respectively. As to main body portion 41, a distal pleat 44c can be looped around main body portion 41 of needle shield 40 to prevent tether 44 from being removed from main body portion 41 during use. See FIG. 5. Although it is preferred to have tether 44 connected to needle hub 34 and needle shield 40 as described above, it is to be understood that other well-known methods for joining two pieces together may be used.

The use of the device is as follows: In order to place catheter 21 into a patient's blood vessel, the clinician substantially longitudinally aligns introducer needle 31 and catheter 21 with the target blood vessel. The bevel defining the sharp distal tip of introducer needle 31 should be facing substantially away from the skin surface during venipuncture. The clinician inserts introducer needle 31 and catheter 21 at a shallow angle, preferably less than about 35 degrees, into the skin so that the sharp distal tip of introducer needle 31 enters the target blood vessel. The clinician then preferably observes a blood flashback in the flashback chamber or near the tip of the catheter at the notch in the needle.

After confirming placement of introducer needle 31 and catheter 21 in the target blood vessel, the clinician advances catheter 21 distally axially along introducer needle 31 into position in the blood vessel. After proper placement of catheter 21 is achieved, the clinician places a finger from her other hand on the patient's skin over the blood vessel distal of the distal end of catheter 21. and the sharp distal tip of introducer needle 31. By placing her finger on the patient's skin and applying sufficient pressure on the skin, the clinician thereby minimizes blood flow through catheter 21. The clinician then withdraws introducer needle 31 from catheter 21 by pulling needle hub 34 in a proximal direction, thereby deploying tether 44. Once sharp distal tip of introducer needle 31 is located within main body portion 41 of needle shield 40, continued proximal movement of needle hub 34 will result in a force sufficient to overcome the force holding fingers 47 to catheter adapter 24 so that main body portion 41 can be removed from catheter adapter 24. Thereafter, the clinician may attach any desired fluid-handling device to side port 25, if a ported catheter is used, or to catheter adapter 24, if a straight catheter is used, and commence the planned treatment. Main body portion 41 of needle shield 40 with the sharp distal tip of introducer needle 31 shielded therein may then be disposed of according to the facility's disposal protocol.

Thus, it is seen that an introducer needle assembly having a needle shield is provided that positively connects the needle shield to the introducer needle assembly to prevent the needle shield from being advanced distally past the distal end of the introducer needle, is compact, simple and easy to use, requires no special features or technique to be operative and is ergonomically comfortable for the clinician to use. It will be apparent that the present invention has been described herein with reference to certain preferred or exemplary embodiments. The preferred or exemplary embodiments described herein may be modified, changed, added to, or deviated from without departing from the intent, spirit and scope of the present invention.

## Claims

1. A needle assembly, comprising:
a needle having a proximal end and a distal end;
a needle hub having a distal end and a proximal end and said needle is secured to said needle huh;
a needle shield slidingly disposed about the needle;
a tether securely connecting the needle hub to the needle shield wherein the tether is compressible;
a member disposed on the shield or the hub operably engaged to the tether wherein the member is adapted to maintain the tether in a compressed condition.

2. A needle assembly, comprising:
a needle having a proximal end and a distal end;
a needle hub having a distal end connected to the proximal end of the needle and a proximal end;
a needle shield disposed about the needle,
a tether connecting the needle hub to the needle shield wherein the tether is compressible;
a member disposed on the tether wherein the member is adapted to maintain the tether in a compressed condition.

3. The needle assembly of claim 2 wherein the tether is formed from a plurality of pleats

4. A needle assembly, comprising:
a needle having a proximal end and a distal end;
a needle hub having a distal end connected to the proximal end of the needle and a proximal end;
a needle shield disposed about the needle;
a tether formed from a plurality of pleats, connecting the needle hub to the needle shield, wherein the tether is capable of being folded;
an adhesive is applied to at least a portion of the tether wherein the adhesive resists un-folding of the tether

5. The needle assembly of claim 4 wherein the plurality of pleats each define a hole therein through which the needle extends.

6. The needle assembly of claim 4 wherein the adhesive is applied to at least one edge of the pleats,

7. The needle assembly of claim 4 wherein the adhesive is applied to at least one face or the pleats.

8. The needle assembly of claim 5 wherein the adhesive is applied to at least one edge of the pleats

9. The needle assembly of claim 5 wherein the adhesive is applied to at least one face of the pleats

10. A method of manufactuing a needle assembly, comprising:
providing a needle having a proximal end and a distal end;
providing a needle hub having a distal end connected to the proximal end of the needle and a proximal end;
providing a needle shield disposed about the needle;
providing a tether formed from a plurality of pleats, connecting the needle hub to the needle shield, wherein the tether is capable of being folded;
compressing the tether into a compressed form.
